# EUROPEAN PATENT SPECIFICATION

(11) **EP 1 846 008 B1**
(45) Date of publication and mention of the grant of the patent: **24.04.2013**
(21) Application number: 06734416.8
(22) Date of filing: 06.02.2006
(51) Int. Cl.: A61K 31/74, A61K 47/30, G02C 7/04, G02B 1/04, C08G 77/04

(54) **METHOD FOR THE MITIGATION OF SYMPTOMS OF CONTACT LENS RELATED DRY EYE**
VERFAHREN ZUR ABSCHWÄCHUNG DER SYMPTOME DES DURCH KONTAKTLINSEN VERURSACHTEN TROCKENEN AUGES
PROCEDE D'ATTENUATION DES SYMPTOMES DE L'OEIL SEC LIES AU PORT DE LENTILLES DE CONTACT

(30) Priority: 07.02.2005 US 650656 P
(43) Date of publication of application: 24.10.2007
(73) Proprietor: Johnson and Johnson Vision Care, Inc., Jacksonville, FL 32256 (US)
(72) Inventor: LORENZ, Kathrine, Osborn, Columbus, OH 43220 (US); STEFFEN, Robert, B., Jacksonville Beach, FL 32250 (US); MCCABE, Kevin, P., Jacksonville, FL 32225 (US); COPPER, Lenora, C., Jacksonville, FL 32223 (US)
(74) Representative: Fisher, Adrian John
(86) International application number: PCT/US2006/004097
(87) International publication number: WO 2006/086303

(56) References cited:
- EP-A- 0 940 693
- WO-A-01/70837
- WO-A-97/20852
- US-A1- 2003 125 498
- US-B1- 6 218 503
- US-B1- 6 364 482
- "Premarket Notification VISTAKON (Senofilcon A) Contact Lens; Summary of Safety and Effectiveness"[Online] 24 November 2004 (2004-11-24), XP002387698 Retrieved from the Internet: URL:http://web.archive.org/web/20041124151 511/http://www.fda.gov/cdrh/pdf4/k042275.p df> [retrieved on 2006-06-23]
- KUNZLER J F: "Silicone Hydrogels for Contact Lens Application" TRENDS IN POLYMER SCIENCE, ELSEVIER SCIENCE PUBLISHERS B.V. AMSTERDAM, NL, vol. 4, no. 2, February 1996 (1996-02), pages 52-59, XP004049314 ISSN: 0966-4793
- JNJ VISION: 'Acuvue Oasys with Hydraclear Plus, New Acuvue Oasys with Hydraclear plus helps eyes in dry, challenging environments', [Online] 23 August 2005, pages 1 - 2 Acuvue Press Room Retrieved from the Internet: <URL:http://www.jnjvision.com/oasys_pr08170 5.htm> [retrieved on 2006-06-16]
- JNJ VISION: 'Acuvue Oasys with Hydraclear Plus, Acuvue Oasys with Hydraclear plus receives FDA approval for extended wear', [Online] 21 December 2005, pages 1 - 2 Acuvue Press Room Retrieved from the Internet: <URL:http://www.jnjvision.com/oasys_pr12212 005.htm> [retrieved on 2006-06-16]

## Description

### Field of the invention

The present invention relates to the mitigation of symptoms of contact lens related dry eye.

### Background of the Invention

Soft contact lenses have been available since the 1980s. While there are many people who can successfully wear contact lenses, there are a number of people who can wear contact lenses for only short periods of time due to contact lens related dry eye ("CLRDE"). Symptoms of this disorder include thin and/or unstable tear films, corneal staining and subjective symptoms such as ocular discomfort, buming/stinging and dryness. Contact lens wear may trigger the onset of these symptoms or may exacerbate the symptoms. People with CLRDE generally can comfortably wear contact lenses only for limited periods of time (less than 6 hours and in some cases less than four hours).

There are many eye drops for the treatment of CLRDE. Because the eye drops are readily washed from the eye by blinking and the normal functioning of the eye, they provide only temporary relief and must be frequently reapplied. Currently, there is one contact lens, PROCLEAR^{®}, commercially available from Cooper Vislon, which is FDA approved and marketed for contact lens wearers with CLRDE. However, many patients with CLRDE symptoms who wear PROCLEAR are still unable to comfortably wear their lenses for an entire day.

The premarket notification for ACUVUE OASYS® contact lenses describes contact lenses made from senofilcon A (http://web.archive.org/web/20041124151511/http://www.fda.gov/cdrh/pdf4/k042275.pdf.) WO 01/70837 describes hydrogels formed from silicone containing reaction mixtures comprising a high molecular weight hydrophilic polymer.

WO 97/20852 describes fluorinated siloxane monomeric units for forming silicone hydrogels having reduced modulus.

EP-A-0 940 693 describes silicone hydrogels formed from reaction mixtures comprising specified monofunctional silicones, which provide the hydrogels with desirable moduli.

US 6,364,482 describes difunctional prepolymers containing at least one silicone containing block.

Accordingly, there remains a need in the art for a contact lens which can extend the comfortable wear time of contact lens wearers displaying CLRDE symptoms.

### Summary of the Invention

The present invention relates to a lubricious polymer for use in a method for the mitigation of symptoms of contact lens related dry eye comprising contacting the surface of the eye of a patient in need of relief of contact lens related dry eye with a contact lens comprising a modulus of less than about 120 psi and at least about 5 weight % of at least one lubricious polymer, wherein said lubricious polymer comprises monomers that are soluble when 10 wt% of the monomer is mixed in water at room temperature.

The present invention relates to senofilcon A for use in a method for the mitigation of symptoms of contact lens related dry eye comprising contacting the surface of the eye of a patient in need of relief of contact lens related dry eye with a contact lens comprising senofilcon A.

### Description of the Figures

Figure 1 is an optical schematic showing the major components of a tear interferometer as used In Example 3.
Figure 2 are tear Interferometer images of ACUVUE^{®} ONE DAY contact lenses.
Figure 3 are tear interferometer Images of ACUVUE OASYS^{®} contact lenses.
Figure 4 are tear interferometer images of COOPER PROCLEAR® contact lenses.

### Description of the Invention

As used herein "contact lens related dry eye" ("CLRDE") is a disorder marked by at least one objective clinical symptom and at least one subjective symptom. Clinical symptoms are selected from (a) a tear film break up time ("TFBUT") of less than about 10 seconds in at least one eye; (b) a fluorescein staining score ≥ 3 on a scale of 0-15 in at least one eye; (c) a lissamine green staining score ≥ 3 on a scale of 0-18 in at least one eye; or (d) a tear meniscus grade of 'abnormal ' in at least one eye. Subjective symptoms are determined via patient feedback and include (a) ≥ about 2 hour difference between average daily contact lens wear time and average daily comfortable contact lens wear time and (b) a rating of frequent or constant feelings of dryness, burning, stinging or discomfort during lens wear. CLRDE sign includes both excessive tear evaporation and Non-Sjogren's aqueous tear deficiency. Excessive tear evaporation is a disorder marked by a TFBUT of about 10 seconds or less in at least one eye or a TFBUT of 10 seconds or less in at least one eye as well as conjunctival or corneal staining of about 3 or greater on the NEI scale. Non-Sjogren's aqueous tear deficiency tear meniscus is a disorder marked by a grade of 'abnormal' in at least one eye or a tear meniscus grade of 'abnormal' in at least one eye as well as conjunctival or corneal staining of 3 or greater on the NEI scale.

As used herein "contact lens" includes ophthalmic devices that reside on the eye. The contact lenses can provide optical correction, vision enhancement, wound care, drug delivery, diagnostic functionality or cosmetic enhancement or effect or a combination of these properties. Accordingly, the contact lenses of the present invention can be spherical, toric, bifocal, may contain cosmetic tints, opaque cosmetic patterns, combinations thereof and the like.

It has been surprisingly found that contact lenses comprising a modulus of less than about 120 psi and at least about 5% of at least one lubricious polymer provide superior comfort than ACUVUE^{®} ONE DAY brand contact lenses to people with CLRDE. The contact lenses of the present invention display superior overall comfort throughout wear, and at the end of the day. The lenses of the present invention were found, in clinical trials, to be significantly more comfortable than ACUVUE® ONE DAY brand contact lenses, which is recognized in the industry as lenses which are among the most comfortable commercially available lenses. The lenses of the present invention were also found to be significantly more comfortable during computer usage. Wearers also reported a significant preference for the lenses of the present invention compared to ACUVUE^{®} ONE DAY brand contact lenses when asked which lenses made their eyes feel moist. By significant, we mean a preference rating of at least 1.5 to 1 in a double masked, clinical trial with at least about 20 patients completing the trial and wearing lenses for at least 8 hours per day in a daily disposable modality for at least one week. End of day comfort data was collected at the end of the week of wear and at least 8 hours after lens insertion. The questionnaires allowed participants the following choices: preferred the test lens, preferred the control lens, preferred both lenses or preferred neither lens. Ratings were generated using all responses indicating a preference between the lenses. ACUVUE® ONE DAY brand contact lenses are soft hydrogel contact lenses made from etafilcon A and commercially available from Johnson & Johnson Vision Care, Inc.

Applicants believe that the contact lenses of the present invention provide superior comfort than ACUVUE^{®} ONE DAY brand contact lenses in people having CLRDE because they support a stable tear film when viewed via a tear interferometer.

Tear film interferometry is based upon well-established optical principles. Tear interferometry uses specular reflectance to generate visible interference patterns which give detailed information of the topography of the tear layer overlaying a contact lens. The intereference patterns are created by using two images of the same source. Specifically, two interfering beams are used, the reflected beam from the front surface of the tear film and the reflected beam from the rear surface of the tear film. The two beams originate from the same lights source 1. When the two reflected beams are combined at a common focus, the result is a series of light and dark interference fringes. These fringes are analogous to the contour lines on a topographical map and the contour interval constant in this case is dependent upon the wavelength of the incident light. Each fringe represents a location on the tear film of consistent film thickness. Moving to an adjacent line or fringe will represent a change in tear film thickness equal to the contour interval. The configuration of the tear interferometer used in the examples is described in Example 3. General descriptions of tear interferometry may also be found in US 4,747,683 and Optometry and Vision Science, M. Doane, Vol. 66, No. 6 pages 383-388.

By stable tear film we mean a tear film of at least moderate thickness and displaying a break up time of at least about 3 seconds when viewed with a tear interferometer. Generally, the thickness of the tear film may be evaluated by the number of fringes visible, the more fringes visible via tear interferometry, the thicker the tear film over the lens. If few fringes, or partial fringes are seen, that usually indicates a very thin tear film and a poorly wetting contact lens in that individual's eye. Rapidly moving fringes indicate that the tear film is rapidly thinning.

It is believed that the incorporation of at least one lubricious polymer provides the lenses of the present invention with the unique stable tear film and smoothness upon dehydration observed via tear interferometry. Suitable lubricious polymers include non-reactive hydrophilic polymers which may be incorporated into the monomer mixture to form an interpenetrating network and reactive hydrophilic polymers. Suitable lubricious polymers have a weight average molecular weight of at least about 50,000 Daltons, and in some embodiments greater than about 100,000 Daltons. The molecular weight of the lubricious polymers may be determined via gel permeation chromatography (GPC) using a ViscoGEL GMPWXL Column with a 20/80 methanol/water ratio with a flow rate 1.0 ml/min. at 30°C.

In addition to the average molecular weight specified above, lubricious polymers also possess, when polymerized and crosslinked to minor amount, a water content of at least about 70%, preferably at least about 80%. For lubricious polymers which are free radical reactive, a "minor amount" of crosslinking may be effected by polymerizing the monomer(s) from which the polymer is formed with a small amount (such as about 7.5 mmol/100 gram of polymer) of crosslinker (for example, EGDMA). Methods for forming crosslinked polymers which are not free radical reactive will be apparent to those of skill in the art from the disclosure contained herein.

Alternatively, the suitability of a polymer for use as a lubricious polymer may be determined by mixing 10 wt% of the monomer from which the polymer is formed in water at room temperature. Monomers that are soluble under these conditions may be used to form lubricious polymers for use in the contact lenses of the present invention. Specific examples of lubricious polymers include high molecular weight hydrophilic polymers and copolymers of polyamides, polylactones, polyimides, polylactams and functionalized polyamides, polylactones, polyimides, polylactams, such as DMA functionalized by copolymerizing DMA with a lesser molar amount of a hydroxyl-functional monomer such as HEMA, and then reacting the hydroxyl groups of the resulting copolymer with materials containing radical polymerizable groups, such as isocyanatoethylmethacrylate or methacryloyl chloride. Hydrophilic polymers or prepolymers made from DMA or n-vinyl pyrrolidone with glycidyl methacrylate may also be used. The glycidyl methacrylate ring can be opened to give a diol which may be used in conjunction with other hydrophilic prepolymers in a mixed system. Specific examples of lubricious polymers include but are not limited to poly-N-vinyl pyrrolidone, poly(N-vinyl-N-methylacetamide), poly-N-vinyl-2- piperidone, poly-N-vinyl-2-caprolactam, poly-N-vinyl-3-methyl-2- caprolactam, poly-N-vinyl-3-methyl-2-piperidone, poly-N-vinyl-4-methyl-2- piperidone, poly-N-vinyl-4-methyl-2-caprolactam, poly-N-vinyl-3-ethyl-2- pyrrolidone, and poly-N-vinyl-4,5-dimethyl-2-pyrrolidone, polyvinylimidazole, poly-N-N-dimethylacrylamide, polyvinyl alcohol, polyethylene oxide, poly 2 ethyl oxazoline, heparin polysaccharides, polysaccharides, mixtures and copolymers (including block or random, branched, multichain, comb-shaped or star shaped) thereof where poly-N-vinylpyrrolidone (PVP), poly(N-vinyl-N-methylacetamide) (PVMA) are particularly preferred. Copolymers might also be used such as graft copolymers of PVP or amphiphilic copolymers having hydrophilic and hydrophobic blocks such as those disclosed in US 10/954,560. The lubricious polymer may be incorporated into the lens polymer without chemical bonding, such as is disclosed in US2003/162,862 and US2003/125,498 or may be copolymerized into the lens matrix or coated onto the contact lens, by any known method such as premold spin casting, as disclosed, for example, in US2003/052,424, grafting, soaking the lens in a polymeric solution as disclosed in US2002/006,521 and US 6,478,423, and the like.

When the lubricious polymer is incorporated into the lens polymer, the lubricious polymer may also comprise polyacrylic acid. However, when the lubricious polymer is coated onto the lens, the lubricious polymer is not polyacrylic acid or poly(N,N-dimethylacrylamide).

Alternatively, the lubricious polymer may be a reactive polymer having a molecular weight as low as 2000. Suitable low molecular weight polymers are disclosed in US Ser. No. 10/954559.

In yet another embodiment, precursors of the lubricious polymers, such as the monomers from which they are formed, may be incorporated into the monomer mix, which is then cured using conditions to form lubricious polymers having the molecular weights disclosed above.

The lenses of the present invention may be made from any known lens materials so long as the lens comprises at least about 5% of at least one lubricious polymer and a modulus of less than about 120 psi. In some embodiments the lens comprises between about 5 and about 20 weight% lubricious polymer, based upon all the components used to make the lens.

The modulus is less than about 120 psi (827 KPa), in some embodiments less than about 100 psi, and in some embodiments between about 40 and 100 psi.

Suitable lens materials include hydrophilic components, silicone containing components and combinations thereof.

The term components includes monomers, macromers and prepolymers. "Monomer" refers to lower molecular weight compounds that can be polymerized to higher molecular weight compounds, polymers, macromers, or prepolymers. The term "macromer" as used herein refers to a high molecular weight polymerizable compound. Prepolymers are partially polymerized monomers or monomers which are capable of further polymerization.

A "silicone-containing component" is one that contains at least two [-Si-O-] repeating units in a monomer, macromer or prepolymer. Preferably, the total Si and attached O are present in the silicone-containing component in an amount greater than 20 weight percent, and more preferably greater than 30 weight percent of the total molecular weight of the silicone-containing component. Useful silicone-containing components preferably comprise polymerizable functional groups such as acrylate, methacrylate, acrylamide, methacrylamide, N-vinyl lactam, N-vinylamide, and styryl functional groups. Examples of silicone-containing components which are useful in this invention may be found in U.S. Pat. Nos. 3,808,178; 4,120,570; 4,136,250; 4,153,641; 4,740,533; 5,034,461 and 5,070,215, and EP080539. These references disclose many examples of olefinic silicone-containing components.

While almost any silicone containing component may be included, in order to provide the lenses of the present invention with the desired modulus, the majority of the mass fraction of the silicone components used in the lens formulation should contain only one polymerizable functional group ("monofunctional silicone containing component"). In silicone containing lenses, to insure the desired balance of oxygen transmissibility and modulus it is preferred that all components having more than one polymerizable functional groups ("multifunctional components") make up no more than 10 mmol/100 g of the reactive components, and preferably no more than 7 memol/100 g of the reactive components. Suitable monofunctional silicone containing components include polysiloxanylalkyl(meth)acrylic monomers of Formula I: 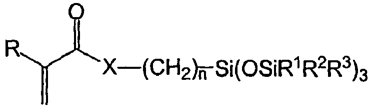
wherein: R denotes H or lower alkyl; X denotes O or NR⁴; each R⁴ independently denotes hydrogen or methyl,
each R¹-R³ independently denotes a lower alkyl radical or a phenyl radical, and
n is 1 or 3 to 10.

Mono-functional polydimethylsiloxanes (mPDMS) may also be used. Suitable mPDMS compounds include Structure II: 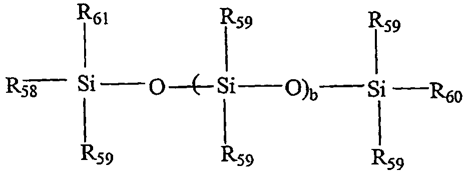 where b = 0 to 100, where it is understood that b is a distribution having a mode equal to a stated value, preferably 4 to 16, more preferably 8 to 10; R₅₈ is a monovalent group containing at least one ethylenically unsaturated moiety, preferably a monovalent group containing a styryl, vinyl, or methacrylate moiety, more preferably a methacrylate moiety; each R₅₉ is independently a monovalent alkyl, or aryl group, which may be further substituted with alcohol, amine, ketone, carboxylic acid or ether groups, preferably unsubstituted monovalent alkyl or aryl groups, more preferably methyl; R₆₀ is a monovalent alkyl, or aryl group, which may be further substituted with alcohol, amine, ketone, carboxylic acid or ether groups, preferably unsubstituted monovalent alkyl or aryl groups, preferably a C₁₋₁₀ aliphatic or aromatic group which may include hetero atoms, more preferably C₃₋₈ alkyl groups, most preferably butyl; and R₆₁ is independently alkyl or aromatic, preferably ethyl, methyl, benzyl, phenyl, or a monovalent siloxane chain comprising from 1 to 100 repeating Si-O units. Examples of suitable mPDMS compounds include 3-methacryloxy-2-hydroxypropyloxy)propylbis(trimethylsiloxy)methylsilane, monomethacryloxypropyl terminated mono-n-butyl terminated polydimethylsiloxane., methacryloxypropylpentamethyl disiloxane, combinations thereof and the like.

Examples of polysiloxanylalkyl (meth)acrylic monomers include methacryloxypropyl tris(trimethylsiloxy) silane, pentamethyldisiloxanyl methylmethacrylate, and methyldi(trimethylsiloxy)methacryloxymethyl silane. Methacryloxypropyl tris(trimethylsiloxy)silane is the most preferred.

In some embodiments monofunctional polydimethylsiloxanes may be preferred, as they lower not only modulus, but also tan δ, while bulky silicones, such as those containing at least one branching trimethylsiloxy group will increase tan δ. Accordingly, at least about 30 and preferably at least about 60 weight% of all the silicone components should be non-bulky silicone containing compounds such as polydimethylsiloxanes.

In one embodiment, where a silicone hydrogel lens is desired, the lens of the present invention will be made from a reactive mixture comprising at least about 20 and preferably between about 20 and 70%wt silicone containing components based on total weight of reactive monomer components from which the polymer is made.

In addition to the monofunctional silicone containing components, multifunctional silicone containing components and/or bulky silicone containing compounds may also be included in amounts which do not impart an undesirably high modulus and/or tan δ.

One class of silicone-containing components is a poly(organosiloxane) prepolymer represented by formula III: 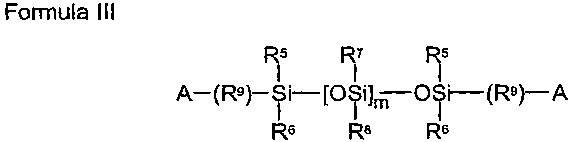 wherein each A independently denotes an activated unsaturated group, such as an ester or amide of an acrylic or a methacrylic acid or an alkyl or aryl group (providing that at least one A comprises an activated unsaturated group capable of undergoing radical polymerization); each of R⁵, R⁶, R⁷ and R⁸ are independently selected from the group consisting of a monovalent hydrocarbon radical or a halogen substituted monovalent hydrocarbon radical having 1 to 18 carbon atoms which may have ether linkages between carbon atoms;

R⁹ denotes a divalent hydrocarbon radical having from 1 to 22 carbon atoms, and

m is 0 or an integer greater than or equal to 1, and preferable 5 to 400, and more preferably 10 to 300. One specific example is α, ω-bismethacryloxypropyl polydimethylsiloxane.

Another useful class of silicone containing components includes silicone containing vinyl carbonate or vinyl carbamate monomers of the following formula: 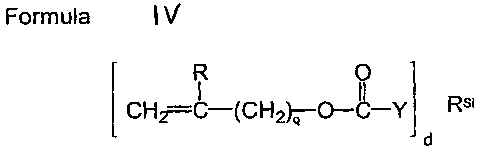 wherein: Y denotes O, S. or NH; R*^{Si}* denotes a silicone-containing organic radical; R denotes
hydrogen or methyl; d is 1, 2, 3 or 4; and q is 0 or 1. Suitable silicone-containing organic radicals R*^{S¡}* include the following:

————(CH₂)_{q}·Si[(CH₂)ₛCH₃]₃

————(CH₂)_{q}·Si[OSi((Ch₂)ₛCH₃)₃]₃

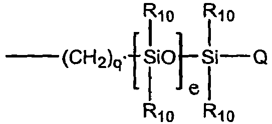

wherein:
Q denotes

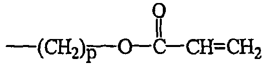

Wherein p is 1 to 6; R¹⁰ denotes an alkyl radical or a fluoroalkyl radical having 1 to 6 carbon atoms; e is 0 to 200; q' is 1, 2, 3 or 4; and s is 0, 1, 2, 3, 4 or 5.

The silicone-containing vinyl carbonate or vinyl carbamate monomers specifically include: 1,3-bis[4-(vinyloxycarbonyloxy)but-1-yl]tetramethyldisiloxane; 3-(vinyloxycarbonylthio) propyl-[tris (trimethylsiloxy)silane]; 3-[tris(trimethylsiloxy)silyl] propyl allyl carbamate; 3-[tris(trimethylsiloxy)silyl] propyl vinyl carbamate; trimethylsilylethyl vinyl carbonate; trimethylsilylmethyl vinyl carbonate, and

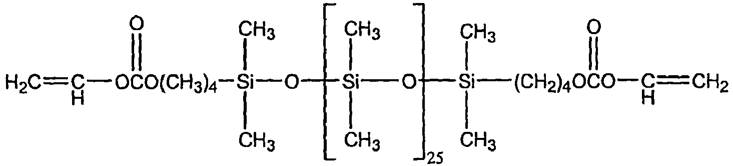

Another class of silicone-containing components includes polyurethane macromers of the following formulae:
Formulae V-VII
   (*D*A*D*G)_{a *}D_{*}D_{*}E¹;
   E(*D*G*D*A)*ₐ* *D*G*D*E¹ or;
   E(*D*A*D*G)*ₐ* *D*A*D*E¹
wherein:
   D denotes an alkyl diradical, an alkyl cycloalkyl diradical, a cycloalkyl diradical, an aryl diradical or an alkylaryl diradical having 6 to 30 carbon atoms,
   G denotes an alkyl diradical, a cycloalkyl diradical, an alkyl cycloalkyl diradical, an aryl diradical or an alkylaryl diradical having 1 to 40 carbon atoms and which may contain ether, thio or amine linkages in the main chain;
   * denotes a urethane or ureido linkage;
   *ₐ* is at least 1;

A denotes a divalent polymeric radical of formula:

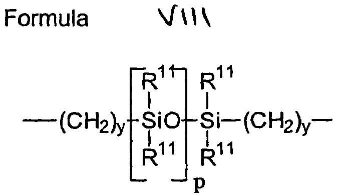

R¹¹ independently denotes an alkyl or fluoro-substituted alkyl group having 1 to10 carbon atoms which may contain ether linkages between carbon atoms; y is at least 1; and p provides a moiety weight of 400 to 10,000; each of E and E¹ independently denotes a polymerizable unsaturated organic radical represented by formula: 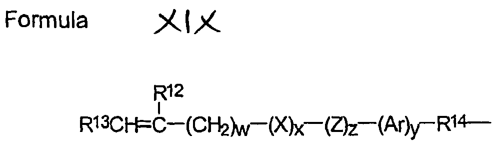 wherein: R¹² is hydrogen or methyl; R¹³ is hydrogen, an alkyl radical having 1 to 6 carbon atoms, or a -CO-Y-R¹⁵ radical wherein Y is -O-,Y-S- or-NH-; R¹⁴ is a divalent radical having 1 to 12 carbon atoms; X denotes -CO- or -OCO-; Z denotes -O- or -NH-; Ar denotes an aromatic radical having 6 to 30 carbon atoms; w is 0 to 6; x is 0 or 1; y is 0 or 1; and z is 0 or 1.

A preferred silicone-containing component is a polyurethane macromer represented by the following formula: 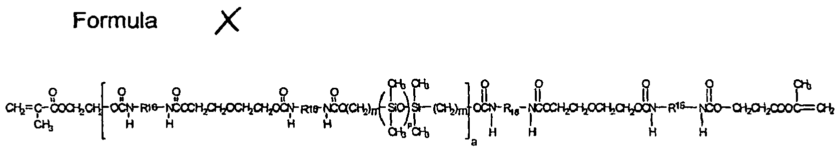 wherein R¹⁶ is a diradical of a diisocyanate after removal of the isocyanate group, such as the diradical of isophorone diisocyanate. Another suitable silicone containing macromer is compound of formula X (in which x + y is a number in the range of 10 to 30) formed by the reaction of fluoroether, hydroxy-terminated polydimethylsiloxane, isophorone diisocyanate and isocyanatoethylmethacrylate.

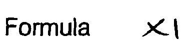

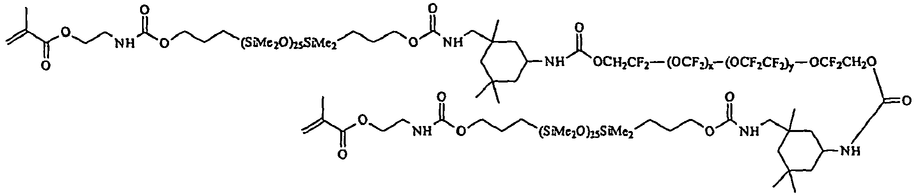

Other silicone containing components suitable for use in this invention include those described is WO 96/31792 such as macromers containing polysiloxane, polyalkylene ether, diisocyanate, polyfluorinated hydrocarbon, polyfluorinated ether and polysaccharide groups. U.S. Pat. Nos. 5,321,108; 5,387,662 and 5,539,016 describe polysiloxanes with a polar fluorinated graft or side group having a hydrogen atom attached to a terminal difluoro-substituted carbon atom. US 2002/0016383 describe hydrophilic siloxanyl methacrylates containing ether and siloxanyl linkanges and crosslinkable monomers containing polyether and polysiloxanyl groups. Any of the foregoing polysiloxanes can also be used as the silicone containing component in this invention.

Hydrophilic monomers are also included in the reactive components used to make the contact lenses of the present invention. The hydrophilic monomers used to make the contact lenses of this invention can be any of the known hydrophilic monomers disclosed in the prior art to make hydrogels.

The preferred hydrophilic monomers used to make the polymer of this invention may be either acrylic- or vinyl-containing. Such hydrophilic monomers may themselves be used as crosslinking agents, however, where hydrophilic monomers having more than one polymerizable functional group are used, their concentration should be limited as discussed above to provide a contact lens having the desired modulus. The term "vinyl-type" or "vinyl-containing" monomers refer to monomers containing the vinyl grouping (-CH=CH₂) and are generally highly reactive. Such hydrophilic vinyl-containing monomers are known to polymerize relatively easily.

"Acrylic-type" or "acrylic-containing" monomers are those monomers containing the acrylic group: (CH₂=CRCOX) wherein R is H or CH₃, and X is O or N, which are also known to polymerize readily, such as N,N-dimethyl acrylamide (DMA), 2-hydroxyethyl methacrylate (HEMA), glycerol methacrylate, 2-hydroxyethyl methacrylamide, polyethyleneglycol monomethacrylate, methacrylic acid and acrylic acid.

Hydrophilic vinyl-containing monomers which may be incorporated into the silicone hydrogels of the present invention include monomers such as N-vinyl amides, N-vinyl lactams (e.g. NVP), N-vinyl-N-methyl acetamide, N-vinyl-N-ethyl acetamide, N-vinyl-N-ethyl formamide, N-vinyl formamide, with NVP being preferred.

Other hydrophilic monomers that can be employed in the invention include polyoxyethylene polyols having one or more of the terminal hydroxyl groups replaced with a functional group containing a polymerizable double bond. Examples include polyethylene glycol, ethoxylated alkyl glucoside, and ethoxylated bisphenol A reacted with one or more molar equivalents of an endcapping group such as isocyanatoethyl methacrylate ("IEM"), methacrylic anhydride, methacryloyl chloride, vinylbenzoyl chloride, or the like, to produce a polyethylene polyol having one or more terminal polymerizable olefinic groups bonded to the polyethylene polyol through linking moieties such as carbamate or ester groups.

Still further examples are the hydrophilic vinyl carbonate or vinyl carbamate monomers disclosed in U.S. Patents No. 5,070,215, and the hydrophilic oxazolone monomers disclosed in U.S. Patents No. 4,910,277. Other suitable hydrophilic monomers will be apparent to one skilled in the art.

In one embodiment the hydrophilic comprises at least one hydrophilic monomer such as DMA, HEMA, glycerol methacrylate, 2-hydroxyethyl methacrylamide, NVP, N-vinyl-N-methyl acrylamide, polyethyleneglycol monomethacrylate, methacrylic acid and acrylic acid with DMA being the most preferred.

The hydrophilic monomers may be present in a wide range of amounts, depending upon the specific balance of properties desired. For silicone hydrogel lenses, amounts of hydrophilic monomer up to about 50 and preferably between about 5 and about 50 weight %, based upon all components in the reactive components are acceptable. For example, in one embodiment lenses of the present invention comprise a water content of at least about 30%, and in another embodiment between about 30 and about 50%. For these embodiments, the hydrophilic monomer may be included in amounts between about 20 and about 50 weight %.

In another embodiment, the contact lens of the present invention is a hydrophilic lens and is formed from lens components comprising primarily or only hydrophilic components and the lubricious polymer. In these embodiments the hydrophilic components are present in amounts from about 70 to about 95 weight percent, and in some embodiments from about 80 to about 95 weight %.

Additionally contact lenses of the present invention may have water contents of at least about 30%, and preferably between about 30 and about 50%. The contact lenses of the present invention may also display advancing contact angles of less than about 80° and in some embodiments less than about 70° as measured using a Wilhelmy dynamic contact angle balance.

In one embodiment, it has been surprisingly found that contact lenses made from senofilcon A provide relief from CLRDE symptoms when worn by patients with CLRDE. Senofilcon A is a polymer formed from the following reactive components: 3-methacryloxy-2-hydroxypropyloxy)propylbis(trimethylsiloxy)methylsilane (SiMAA), monomethacryloxypropyl terminated mono-n-butyl terminated polydimethylsiloxane (mPDMS), N,N-dimethyl acrylamide (DMA), 2-hydroxyethyl methacrylate (HEMA), poly-N-vinyl pyrrolidone (PVP), a UV absorber and a visibility tint.

The contact lenses of the present invention may be used for the temporary relief or mitigation of subjective CLRDE symptoms such as dryness, burning, stinging and discomfort. Relief or mitigation of symptoms may be clinically measured via a clinical study. For example, in the study patients wear a control lenses and the senofilcon A lenses for a predetermined period of time and are asked about CLRDE symptoms such as ocular discomfort, dryness, burning and stinging. If the control lens is a lens approved for use for the treatment of CLRDE, like Proclear Compatibles, the test lens would only need subjective scores equivalent to Proclear Compatibles to be considered useful for the relief or mitigation of CLRDE symptoms. If the control lens is a conventional lens, the test lens would need subjective scores better than the control to be considered useful for the relief or mitigation of CLRDE symptoms. Accordingly, as used herein, temporary relief or mitigation of CLRDE symptoms means a clinically measurable dimunition of at least one CLRDE symptom, such as dryness, burning, stinging or discomfort as measured in a masked, clinical trial with at least about 10 patients completing the trial and wearing lenses for at least about 8 hours per day on average for at least one week. Preferably the clinical trial is a double-masked trial with at least about 20 patients completing the trial and wearing lenses for at least about 8 hours on average per day for at least one week.

The duration of contact (or wear time) will vary depending on the individual patient. However, because the symptomatic benefit is provided by the lens itself and not an additive that elutes from the lens, the benefits derived from using the lenses in accordance with the present invention extend throughout the entire wear time. Suitable wear times include at least 8 hours, at least 12 hours, at least 24 hours, at least a week and for some hours, at least 12 hours, at least 24 hours, at least a week and for some patients at least a month of continuous wear.

The lenses may be worn continuously or may be worn only during waking hours. If the lenses are worn during waking hours only, the lenses may be cleaned and stored using any conventional cleaning and/or storing solution.

Tear film break-up (TFBUT) time was measured as follows. Five (5) µl of non-preserved, 2% sodium fluorescein was instilled into the right of a clinical patient using a micropipette. The patient was instructed to blink 2 times without squeezing his/her eyelids together, then stare straight ahead. Immediately after the second blink and within 30 seconds of fluorescein instillation, the right eye was observed through a slit lamp and the time was measured for the first sign of disruption of the tear film. The measurement was immediately repeated on the right eye. If the recorded TFBUT measurements were less than 10 seconds and greater than 2 seconds apart, a third measurement was taken. The measurements were averaged. The procedure was repeated for the left eye.

Fluorescein staining is measured by instilling 5 microliters of 2% fluorescein solution into the inferior conjunctival cul-de-sac. The patient is instructed to blink several times. After 5 minutes the eye is evaluated using a slit lamp. The staining is rated in each of five regions using a 0-3 scale extracted from the 1995 NEI workshop on Dry Eye (Section 11.1) (0 = None, 1 = Discrete, countable, 2 = Moderate, coalescent, Uncountable, 3 = Confluent). The cumulative sum of the regional scores is reported.

Lissamine green staining score is measured by instilling 10 microliters of lissamine green into the inferior cul-de-sac a drop of each eye. The patient is instructed to blink several times. After 5 minutes the eye is evaluated using a slit lamp. The staining is rated in each of six regions using a 0-3 scale extracted from the 1995 NEI workshop on Dry Eye (Section 11.1) (0 = None, 1 = Discrete, countable, 2 = Moderate, coalescent, Uncountable, 3 = Confluent). The cumulative sum of the regional scores is reported.

The tear meniscus is evaluated by observing the eye using a slit lamp and a diffuse/specular reflection illumination source under low to moderate magnification. A normal tear film produced a specular reflection off the upper edge of the lower lid tear prism which appeared to float well above the lower lid as the slit lamp light source is moved from one side to the other. An abnormal tear film (low tear volume) produced a lower lid tear prism reflection which was absent or appeared immediately adjacent to the lid margin. The reflex will followed an irregular path across the lid margin as the light beam was moved from side to side.

### Examples

### Example 1

Senofilcon A lenses were made as disclosed in WO03/022321 (Example 1) and clinically evaluated against ACUVUE^{®} ONE DAY brand contact lenses (etafilcon A). The clinical evaluation was a single masked (patient), randomized, bilateral cross-over study with twenty-eight patients completing the study. Only patients who reported dry eye symptoms with their own contacts using the following eligibility criteria were recruited.
1. The subject, with their own lenses (or their most recent lens wearing experience), must have reported subjective experiences listed in either (a) or (b):
   (a) a score of greater than 40 on a modified McMonnies questionnaire, as disclosed in Guillon M, Allary JC, Guillon JP, Orsborn G: Clinical Management of Regular Replacement: Part 1. Selection of Replacement Frequency. ICLC Vol. 19, May/June 1992 pp104- 120. (where 0=not dry at all; 168= severely dry)
      o Subjective rating of Comfort/Dryness/Grittiness of less than 35 on a scale of 50, (0= very uncomfortable, always dry/constantly gritty and 50= excellent comfort/no dryness/no grittiness) and wearing time < 12 hours; or
      o Used eye drops more than 3 times/day ≥3; or
      o Subjective rating of Comfort/Dryness/Grittiness of less than <25, on the above described scale; or
      o Daily wearing time of less than 8 hours.
   (b) a score of greater than 50 on a modified McMonnies questionnaire; and
      o Subjective rating of Comfort/Dryness/Grittiness of less than 35, on the above described scale; or
      o Daily wearing time of less than 12 hours; or
      o Used eye drops more than 3 times/day.

The lenses were worn in a daily disposable mode (lenses thrown out each night) for a period of one week. After one week of wear for each lens, the patients were asked to rate which lens they preferred for initial comfort, end of day comfort, overall comfort and dryness. Table 1 shows the preference data.

**Table 1**

| Attribute | Ex. 1 v. ACUVUE® ONE DAY |
|---|---|
| | contact lenses |
| Initial Comfort | 15:7 |
| End of Day Comfort | 14:9 |
| Overall Comfort | 15:9 |
| Dryness | 14:10 |

### Example 2

Senofilcon A lenses were made as disclosed in WO03/022321 (Example 1) and clinically evaluated against Proclear Compatibles® (omafilcon A) contact lenses. The clinical evaluation was a double masked, randomized, bilateral cross-over study with 54 patients completing the study. Only patients meeting the following criteria were enrolled:
● reported comfortable contact lens wearing time that is at least 2 hours less than actual wear time,
● or reported at least moderate intensity (3,4,or 5 out of a scale of 0-5) or frequency (3 or 4 out of a scale of 0-4) of at least one of the following: symptoms: comfort, dryness, or burning/stinging;
and have at least one of the following:
● a TFBUT of less than 10 seconds in at least one eye,
● fluorescein staining score of at least 3 on a scale of 0-15 in at least one eye, or
● lissamine green staining score of at least 3 on a scale of 0-18 in at least one eye, or
● a tear miniscus grade of abnormal in at least one eye.

Sixty-three percent (63%) of the enrolled patients were classified as having evaporative tear deficiency and thirty-seven percent (37%) were classified as having both evaporative tear deficiency and aqueous tear deficiency. The lenses were worn in a daily wear mode (nightly removal) for a period of one month using ReNu Multi-Plus Multi-Purpose Solution. Both lens types were replaced after 2 weeks of lens wear. After two and four weeks of wear for each lens, the patients were asked to rate the lenses for ocular discomfort and visual acuity. At each visit the senofilcon A lenses were rated as good or better than the Proclear Compatibles® lenses on both ocular discomfort (0.6 2 weeks and 0.7 after four weeks in the cohort population on a 4 point scale:0=no discomfort, 4 = severe discomfort) and visual acuity (average of 0.0 on logMar scale for both senofilcon A and Proclear Compatibles® at the 4-week follow-up visit).

Those of ordinary skill in the art will recognize that many modifications and variations of the present invention may be implemented. The foregoing description and the following claims are intended to cover all such modifications and variations.

### Example 3

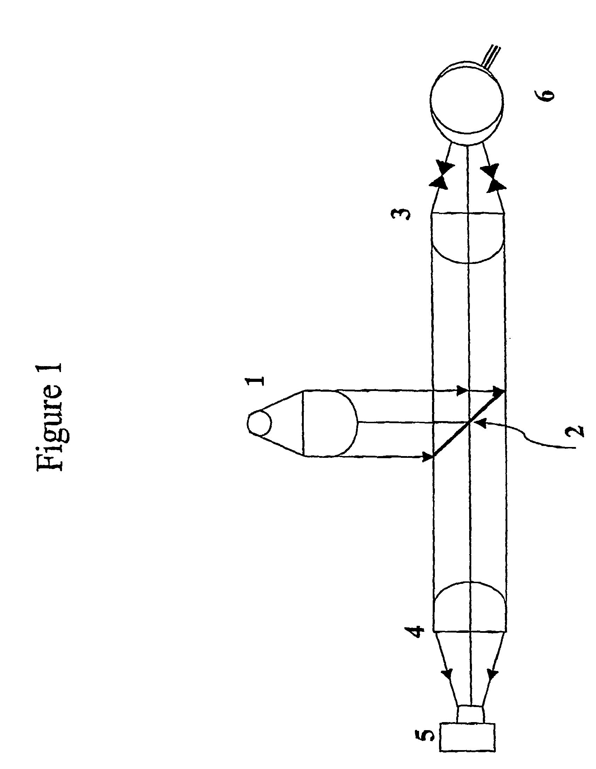

A tear interferometer, as shown in Figure 1 (simplified optical schematic of the instrument) was used to evaluate the tear films on the following commercially available contact lenses ACUVUE ONE DAY, ACUVUE OASYS and BIOCOMPATIBLES PROCLEAR brand contact lenses. The interferometer had the following major components a monochromatic green light source (centered around 5461 Angstroms) 1, a collimated beam formed by a condenser lens system, a beam splitter 2, that reflects about 4% of the beam to the eye (and transmits over 95%), and the objective lens 3, which both converges the light to the center of curvature of the cornea of the eye 6, and collimates the light reflecting back from the tear film. A second, identical lens 4, focuses this collimated returning light onto the detector 5, of a video camera. It can also be used as a direct-viewing instrument. Lamp 1, was set at the desired brightness as most of the direct illumination from the lamp 1 passed through the beamsplitter and was lost before it reached the eye. The objective lenses 3and 4 were identical in design, used in a back-to-back configuration, and optically very fast, having an f-number of 1.2. Since the imaging system is optically symmetrical the on-axis, optical aberrations of the system were minimized.

Ten subjects wore each of lenses listed above for 8-hour periods. All subjects wore each of the three lenses. Interferometric examinations were made on each subject at 30-minutes and 8-hour wearing times. The subjects wore the same lens type in both eyes during each wearing period. During the exams, the subjects were asked to blink normally for about 20-30 seconds, then to keep their lids open for an extended period (10 seconds or more) without blinking. The tear film thickness, time to dry (time it took for the contact lens to become dry when withholding blinks), and recovery (recovery from dryness to a wet surface) were evaluated. Based upon the evaluation the lenses were rated on a scale of 1-5 (5 being the best overall performance). The results are shown in the Table below.

| Lens | TF Thickness | Time to dry | recovery | Rating |
|---|---|---|---|---|
| ONE DAY | moderate | Long | good | 3.5 |
| OASYS | Modest to moderate | Long | good | 4.5 |
| PROCLEAR | thin | Short (<3 sec) | slow | 2 |

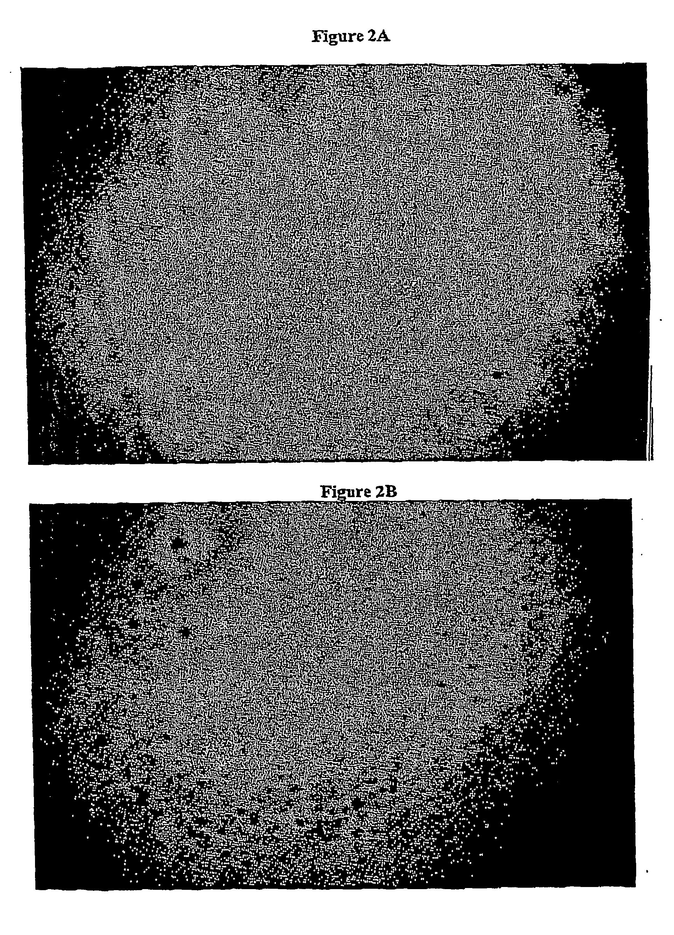

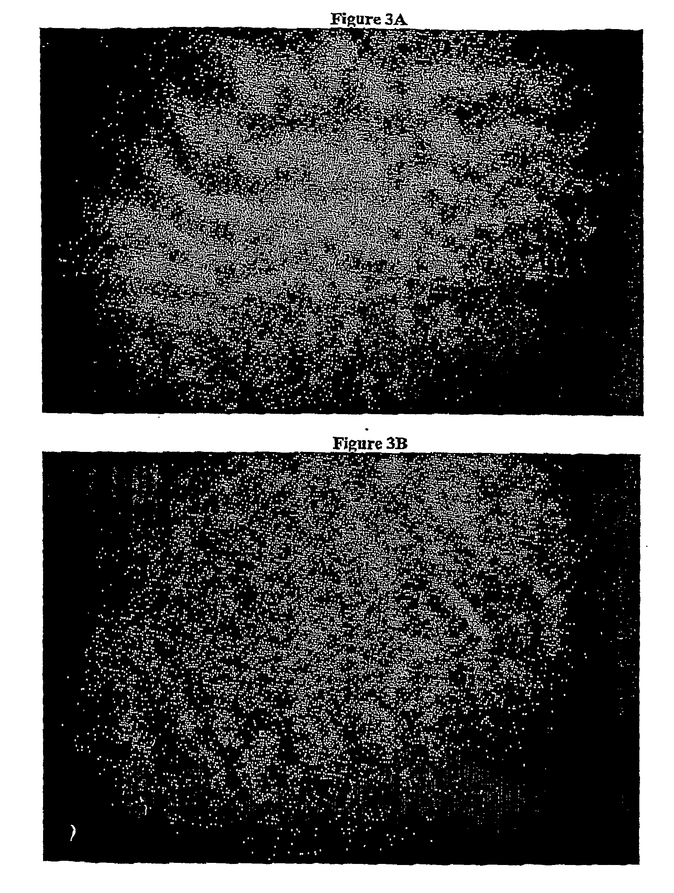

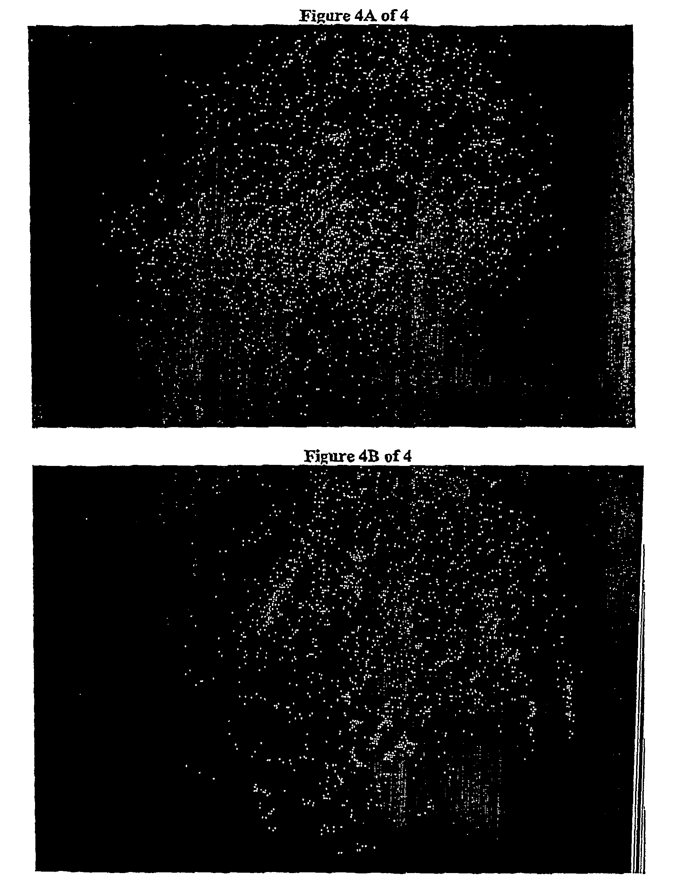

Representative examples of the interferometric images which were collected are shown in Figures 2 through 4. All images shown were recorded after eight hours of wear in the left eye of the same patient. The image on the left of each set ("A") is the image of the tear film taken two seconds after a blink. The image on the right of each set ("B") is the image of the tear film taken ten seconds after a blink. Figures 2A and 3A (ACUVUE® ONE DAY and ACUVUE OASYS®, respectively) show numerous distinct fringes, indicating tear films with moderate thickness. The interferometric pattern for the Cooper PROCLEAR® contact lens (Figure 4A) showed few fringes, which were rapidly moving. Rapidly moving fringes indicates that the tear film is rapidly thinning. The image on the right of Figure 4 shows the PROCLEAR contact lens after 10 seconds without blinking. The image shows several dark pits indicating a "craggy" surface. Several blinks were required to achieve an interferometric image similar to Figure 4A.

Even after 10 seconds the ACUVUE OASYS brand contact lens did not display dark pits or crevasses. A strong fringe pattern was observed after a single blink.

## Claims

1. Senofilcon A for use in a method for the mitigation of symptoms of contact lens related dry eye comprising contacting the surface of the eye of a patient in need of mitigation of symptoms for contact lens related dry eye with a contact lens comprising senofilcon A.

2. Senofilcon A for use in a method for the mitigation of symptoms of contact lens related dry eye according to claim 1 wherein said symptoms include dryness, burning, stinging and discomfort.

3. Senofilcon A for use in a method for the mitigation of symptoms of contact lens related dry eye according to claim 1 wherein said contacting step comprises a wear time of at least 8 hours.

4. Senofilcon A for use in a method for the mitigation of symptoms of contact lens related dry eye according to claim 1 wherein said contacting step comprises a wear time of at least 12 hours.

5. Senofilcon A for use in a method for the mitigation of symptoms of contact lens related dry eye according to claim 1 wherein said contacting step comprises a wear time of at least 24 hours.

6. Senofilcon A for use in a method for the mitigation of symptoms of contact lens related dry eye according to claim 1 wherein said contacting step comprises a wear time of at least 1 week.

7. Senofilcon A for use in a method for the mitigation of symptoms of contact lens related dry eye according to claim 1 wherein said contacting step comprises a wear time of at least 1 month.

8. Senofilcon A for use in a method for the mitigation of symptoms of contact lens related dry eye according to claim 1 wherein said patient is in need of mitigation of symptoms of excessive tear evaporation.

9. Senofilcon A for use in a method for the mitigation of symptoms of contact lens related dry eye according to claim 1 wherein said patient is in need of mitigation of symptoms of Non-Sjogren's aqueous tear deficiency.

10. A lubricious polymer for use in a method for the mitigation of symptoms of contact lens related dry eye comprising contacting the surface of the eye of a patient in need of mitigation of symptoms for contact lens related dry eye with a contact lens comprising a modulus of less than 827 kPa (120 psi) and at least 5% of at least one lubricious polymer, wherein said lubricious polymer comprises monomers that are soluble when 10 wt% of the monomer is mixed in water at room temperature.

11. The lubricious polymer for use in a method for the mitigation of symptoms of contact lens related dry eye according to claim 10 wherein said contact lens comprises at least one silicone containing component.

12. The lubricious polymer for use in a method for the mitigation of symptoms of contact lens related dry eye according to claim 10 wherein said lubricious polymer has a weight average molecular weight of at least 50,000 Daltons.

13. The lubricious polymer for use in a method for the mitigation of symptoms of contact lens related dry eye according to claim 10 wherein said lubricious polymer has a weight average molecular weight of at least 100,000 Daltons.

14. The lubricious polymer for use in a method for the mitigation of symptoms of contact lens related dry eye according to claim 10 wherein said lubricious polymer comprises a water content of at least about 70% when polymerized and crosslinked to minor amount.

15. The lubricious polymer for use in a method for the mitigation of symptoms of contact lens related dry eye according to claim 10 wherein said lubricious polymer is selected from the group consisting of high molecular weight hydrophilic polymers and copolymers of polyamides, polylactones, polyimides, polylactams and functionalized polyamides, polylactones, polyimides and polylactams.

16. The lubricious polymer for use in a method for the mitigation of symptoms of contact lens related dry eye according to claim 10 wherein said lubricious polymer is selected from the group consisting of poly-N-vinyl pyrrolidone, poly(N-vinyl-N-methylacetamide), poly-N-vinyl-2- piperidone, poly-N-vinyl-2-caprolactam, poly- N-vinyl-3-methyl-2- caprolactam, poly-N-vinyl-3-methyl-2-piperidone, poly-N- vinyl-4-methyl-2- piperidone, poly-N-vinyl-4-methyl-2-caprolactam, poly-N-vinyl- 3-ethyl-2-pyrrolidone, and poly-N-vinyl-4,5-dimethyl-2-pyrrolidone, polyvinylimidazole, poly-N-N-dimethylacrylamide, polyvinyl alcohol, polyethylene oxide, poly 2 ethyl oxazoline, heparin polysaccharides, polysaccharides, mixtures and copolymers (including block or random, branched, multichain, comb-shaped or star shaped) thereof.

17. The lubricious polymer for use in a method for the mitigation of symptoms of contact lens related dry eye according to claim 10 wherein said lubricious polymer comprises repeating units derived from N-vinylpyrrolidone, N-vinyl-N-methylacetamide and mixtures thereof.

18. The use of senofilcon A for the manufacture of a contact lens for the mitigation of symptoms of contact lens related dry eye by contacting the surface of the eye of a patient in need of mitigation of symptoms for contact lens related dry eye with said contact lens

19. The use of claim 18 wherein said symptoms include dryness, burning, stinging and discomfort.

20. The use of claim 18 wherein said contacting step comprises a wear time of at least 8 hours.

21. The use of claim 18 wherein said contacting step comprises a wear time of at least 12 hours.

22. The use of claim 18 wherein said contacting step comprises a wear time of at least 24 hours.

23. The use of claim 18 wherein said contacting step comprises a wear time of at least 1 week.

24. The use of claim 18 wherein said contacting step comprises a wear time of at least 1 month.

25. The use of claim 18 wherein said patient is in need of mitigation of symptoms of excessive tear evaporation.

26. The use of claim 18 wherein said patient is in need of mitigation of symptoms of Non-Sjogren's aqueous tear deficiency.

27. The use of a lubricious polymer for the manufacture of a contact lens comprising a modulus of less than 827 kPa (120 psi) and at least 5% of at least one lubricious polymer for the mitigation of symptoms of contact lens related dry eye by contacting the surface of the eye of a patient in need of mitigation of symptoms for contact lens related dry eye with said contact lens wherein said lubricious polymer comprises monomers that are soluble when 10 wt% of the monomer is mixed in water at room temperature.

28. The use of claim 27 wherein said contact lens comprises at least one silicone containing component.

29. The use of claim 27 wherein said lubricious polymer has a weight average molecular weight of at least 50,000 Daltons.

30. The use of claim 27 wherein said lubricious polymer has a weight average molecular weight of at least 100,000 Daltons.

31. The use of claim 27 wherein said lubricious polymer comprises a water content of at least 70% when polymerized and crosslinked to minor amount.

32. The use of claim 27 wherein said lubricious polymer is selected from the group consisting of high molecular weight hydrophilic polymers and copolymers of polyamides, polylactones, polyimides, polylactams and functionalized polyamides, polylactones, polyimides and polylactams.

33. The use of claim 27 wherein said lubricious polymer is selected from the group consisting of poly-N-vinyl pyrrolidone, poly(N-vinyl-N- methylacetamide), poly-N-vinyl-2- piperidone, poly-N-vinyl-2-caprolactam, poly- N-vinyl-3-methyl-2-caprolactam, poly-N-vinyl-3-methyl-2-piperidone, poly-N- vinyl-4-methyl-2-piperidone, poly-N-vinyl-4-methyl-2-caprolactam, poly-N-vinyl- 3-ethyl-2- pyrrolidone, and poly-N-vinyl-4,5-dimethyl-2-pyrrolidone, polyvinylimidazole, poly-N-N-dimethylacrylamide, polyvinyl alcohol, polyethylene oxide, poly 2 ethyl oxazoline, heparin polysaccharides, polysaccharides, mixtures and copolymers (including block or random, branched, multichain, comb-shaped or star shaped) thereof.

34. The use of claim 27 wherein said lubricious polymer comprises repeating units derived from N-vinylpyrrolidone, N-vinyl-N-methylacetamide and mixtures thereof.

## Patentansprüche

1. Senofilcon A für die Verwendung bei einem Verfahren zum Mildern von Symptomen von mit Kontaktlinsen in Beziehung stehendem trockenem Auge, umfassend das Inkontaktbringen der Oberfläche des Auges eines Patienten mit Bedarf an der Milderung von Symptomen von mit Kontaktlinsen in Beziehung stehendem trockenem Auge mit einer Kontaktlinse, die Senofilcon A umfasst.

2. Senofilcon A für die Verwendung bei einem Verfahren zum Mildern von Symptomen von mit Kontaktlinsen in Beziehung stehendem trockenem Auge gemäß Anspruch 1, wobei die Symptome Trockenheit, Brennen, Stechen und Beschwerden umfassen.

3. Senofilcon A für die Verwendung bei einem Verfahren zum Mildern von Symptomen von mit Kontaktlinsen in Beziehung stehendem trockenem Auge gemäß Anspruch 1, wobei der Schritt des Inkontaktbringens eine Tragezeit von wenigstens 8 Stunden umfasst.

4. Senofilcon A für die Verwendung bei einem Verfahren zum Mildern von Symptomen von mit Kontaktlinsen in Beziehung stehendem trockenem Auge gemäß Anspruch 1, wobei der Schritt des Inkontaktbringens eine Tragezeit von wenigstens 12 Stunden umfasst.

5. Senofilcon A für die Verwendung bei einem Verfahren zum Mildern von Symptomen von mit Kontaktlinsen in Beziehung stehendem trockenem Auge gemäß Anspruch 1, wobei der Schritt des Inkontaktbringens eine Tragezeit von wenigstens 24 Stunden umfasst.

6. Senofilcon A für die Verwendung bei einem Verfahren zum Mildern von Symptomen von mit Kontaktlinsen in Beziehung stehendem trockenem Auge gemäß Anspruch 1, wobei der Schritt des Inkontaktbringens eine Tragezeit von wenigstens 1 Woche umfasst.

7. Senofilcon A für die Verwendung bei einem Verfahren zum Mildern von Symptomen von mit Kontaktlinsen in Beziehung stehendem trockenem Auge gemäß Anspruch 1, wobei der Schritt des Inkontaktbringens eine Tragezeit von wenigstens 1 Monat umfasst.

8. Senofilcon A für die Verwendung bei einem Verfahren zum Mildern von Symptomen von mit Kontaktlinsen in Beziehung stehendem trockenem Auge gemäß Anspruch 1, wobei der Patient Bedarf an der Milderung von Symptomen von übermäßiger Tränenverdunstung hat.

9. Senofilcon A für die Verwendung bei einem Verfahren zum Mildern von Symptomen von mit Kontaktlinsen in Beziehung stehendem trockenem Auge gemäß Anspruch 1, wobei der Patient Bedarf an der Milderung von Symptomen von Nicht-Sjögrenwässrige-Tränen-Mangel hat.

10. Gleitfähiges Polymer für die Verwendung bei einem Verfahren zum Mildern von Symptomen von mit Kontaktlinsen in Beziehung stehendem trockenem Auge, umfassend das Inkontaktbringen der Oberfläche des Auges eines Patienten mit Bedarf an der Milderung von Symptomen von mit Kontaktlinsen in Beziehung stehendem trockenem Auge mit einer Kontaktlinse, die einen Modul von weniger als 827 kPa (120 psi) und wenigstens 5 % an wenigstens einem gleitfähigen Polymer umfasst, wobei das gleitfähige Polymer Monomere umfasst, die löslich sind, wenn 10 Gew.-% des Monomers bei Raumtemperatur in Wasser gemischt sind.

11. Gleitfähiges Polymer für die Verwendung bei einem Verfahren zum Mildern von Symptomen von mit Kontaktlinsen in Beziehung stehendem trockenem Auge gemäß Anspruch 10, wobei die Kontaktlinse wenigstens eine Silicon-enthaltende Komponente umfasst.

12. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer ein gewichtsgemitteltes Molekulargewicht von wenigstens 50.000 Dalton aufweist.

13. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer ein gewichtsgemitteltes Molekulargewicht von wenigstens 100.000 Dalton aufweist.

14. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer einen Wassergehalt von wenigstens etwa 70 %, wenn polymerisiert und zu einem geringen Grad vernetzt, umfasst.

15. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer ausgewählt ist aus der Gruppe bestehend aus hochmolekularen hydrophilen Polymeren und Copolymeren von Polyamiden, Polylactonen, Polyimiden, Polylactamen und funktionalisierten Polyamiden, Polylactonen, Polyimiden und Polylactamen.

16. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer ausgewählt ist aus der Gruppe bestehend aus Poly-N-vinylpyrrolidon, Poly(N-vinyl-N-methylacetamid), Poly-N-vinyl-2-piperidon, Poly-N-vinyl-2-caprolactam, Poly-N-vinyl-3-methyl-2-caprolactam, Poly-N-vinyl-3-methyl-2-piperidon, Poly-N-vinyl-4-methyl-2-piperidon, Poly-N-vinyl-4-methyl-2-caprolactam, Poly-N-vinyl-3-ethyl-2-pyrrolidon und Poly-N-vinyl-4,5-dimethyl-2-pyrrolidon, Polyvinylimidazol, Poly-N-N-dimethylacrylamid, Polyvinylalkohol, Polyethylenoxid, Poly-2-ethyloxazolin, Heparinpolysacchariden, Polysacchariden, Gemischen und Copolymeren (einschließlich Block- oder Zufalls-, verzweigten, Multiketten-, kammartigen oder sternförmigen) davon.

17. Gleitfähiges Polymer für die Verwendung bei einem Verfahren zum Mildern von Symptomen von mit Kontaktlinsen in Beziehung stehendem trockenem Auge gemäß Anspruch 10, wobei das gleitfähige Polymer Wiederholungseinheiten, abgeleitet von N-Vinylpyrrolidon, N-Vinyl-N-methylacetamid und Gemischen davon, umfasst.

18. Verwendung von Senofilcon A für die Herstellung einer Kontaktlinse zum Mildern von Symptomen von mit Kontaktlinsen in Beziehung stehendem trockenem Auge durch in Kontakt bringen der Oberfläche des Auges eines Patienten mit Bedarf an der Milderung von Symptomen von mit Kontaktlinsen in Beziehung stehendem trockenem Auge mit der Kontaktlinse.

19. Verwendung gemäß Anspruch 18, wobei die Symptome Trockenheit, Brennen, Stechen und Beschwerden umfassen.

20. Verwendung gemäß Anspruch 18, wobei der Schritt des Inkontaktbringens eine Tragezeit von wenigstens 8 Stunden umfasst.

21. Verwendung gemäß Anspruch 18, wobei der Schritt des Inkontaktbringens eine Tragezeit von wenigstens 12 Stunden umfasst.

22. Verwendung gemäß Anspruch 18, wobei der Schritt des Inkontaktbringens eine Tragezeit von wenigstens 24 Stunden umfasst.

23. Verwendung gemäß Anspruch 18, wobei der Schritt des Inkontaktbringens eine Tragezeit von wenigstens 1 Woche umfasst.

24. Verwendung gemäß Anspruch 18, wobei der Schritt des Inkontaktbringens eine Tragezeit von wenigstens 1 Monat umfasst.

25. Verwendung gemäß Anspruch 18, wobei der Patient Bedarf an der Milderung von Symptomen von übermäßiger Tränenverdunstung hat.

26. Verwendung gemäß Anspruch 18, wobei der Patient Bedarf an der Milderung von Symptomen von Nicht-Sjögren-wässrige-Tränen-Mangel hat.

27. Verwendung eines gleitfähigen Polymers für die Herstellung einer Kontaktlinse, die einen Modul von weniger als 827 kPa (120 psi) und wenigstens 5 % an wenigstens einem gleitfähigen Polymer umfasst, für die Milderung von Symptomen von mit Kontaktlinsen in Beziehung stehendem trockenem Auge durch Inkontaktbringen der Oberfläche des Auges eines Patienten mit Bedarf an der Milderung von Symptomen von mit Kontaktlinsen in Beziehung stehendem trockenem Auge mit der Kontaktlinse, wobei das gleitfähige Polymer Monomere umfasst, die löslich sind, wenn 10 Gew.-% des Monomers bei Raumtemperatur in Wasser gemischt sind.

28. Verwendung gemäß Anspruch 27, wobei die Kontaktlinse wenigstens eine Silicon-enthaltende Komponente umfasst.

29. Verwendung gemäß Anspruch 27, wobei das gleitfähige Polymer ein gewichtsgemitteltes Molekulargewicht von wenigstens 50.000 Dalton aufweist.

30. Verwendung gemäß Anspruch 27, wobei das gleitfähige Polymer ein gewichtsgemitteltes Molekulargewicht von wenigstens 100.000 Dalton aufweist.

31. Verwendung gemäß Anspruch 27, wobei das gleitfähige Polymer einen Wassergehalt von wenigstens etwa 70 %, wenn polymerisiert und zu einem geringen Grad vernetzt, umfasst.

32. Verwendung gemäß Anspruch 27, wobei das gleitfähige Polymer ausgewählt ist aus der Gruppe bestehend aus hochmolekularen hydrophilen Polymeren und Copolymeren von Polyamiden, Polylactonen, Polyimiden, Polylactamen und funktionalisierten Polyamiden, Polylactonen, Polyimiden und Polylactamen.

33. Verwendung gemäß Anspruch 27, wobei das gleitfähige Polymer ausgewählt ist aus der Gruppe bestehend aus Poly-N-vinylpyrrolidon, Poly(N-vinyl-N-methylacetamid), Poly-N-vinyl-2-piperidon, Poly-N-vinyl-2-caprolactam, Poly-N-vinyl-3-methyl-2-caprolactam, Poly-N-vinyl-3-methyl-2-piperidon, Poly-N-vinyl-4-methyl-2-piperidon, Poly-N-vinyl-4-methyl-2-caprolactam, Poly-N-vinyl-3-ethyl-2-pyrrolidon und Poly-N-vinyl-4,5-dimethyl-2-pyrrolidon, Polyvinylimidazol, Poly-N-N-dimethylacrylamid, Polyvinylalkohol, Polyethylenoxid, Poly-2-ethyloxazolin, Heparinpolysacchariden, Polysacchariden, Gemischen und Copolymeren (einschließlich Block- oder Zufalls-, verzweigten, Multiketten-, kammartigen oder sternförmigen) davon.

34. Verwendung gemäß Anspruch 27, wobei das gleitfähige Polymer Wiederholungseinheiten, abgeleitet von N-Vinylpyrrolidon, N-Vinyl-N-methylacetamid und Gemischen davon, umfasst.

## Revendications

1. Senofilcon A pour utilisation dans un procédé pour l'atténuation de symptômes de sécheresse oculaire associée aux lentilles de contact comprenant la mise en contact de la surface de l'oeil d'un patient nécessitant l'atténuation de symptômes pour la sécheresse oculaire associée aux lentilles de contact avec une lentille de contact comprenant Senofilcon A.

2. Senofilcon A pour utilisation dans un procédé pour l'atténuation de symptômes de sécheresse oculaire associée aux lentilles de contact selon la revendication 1, lesdits symptômes comprenant sécheresse, brûlure, piqûre et gêne.

3. Senofilcon A pour utilisation dans un procédé pour l'atténuation de symptômes de sécheresse oculaire associée aux lentilles de contact selon la revendication 1, ladite étape de mise en contact comprenant un temps de port d'au moins 8 heures.

4. Senofilcon A pour utilisation dans un procédé pour l'atténuation de symptômes de sécheresse oculaire associée aux lentilles de contact selon la revendication 1, ladite étape de mise en contact comprenant un temps de port d'au moins 12 heures.

5. Senofilcon A pour utilisation dans un procédé pour l'atténuation de symptômes de sécheresse oculaire associée aux lentilles de contact selon la revendication 1, ladite étape de mise en contact comprenant un temps de port d'au moins 24 heures.

6. Senofilcon A pour utilisation dans un procédé pour l'atténuation de symptômes de sécheresse oculaire associée aux lentilles de contact selon la revendication 1, ladite étape de mise en contact comprenant un temps de port d'au moins 1 semaine.

7. Senofilcon A pour utilisation dans un procédé pour l'atténuation de symptômes de sécheresse oculaire associée aux lentilles de contact selon la revendication 1, ladite étape de mise en contact comprenant un temps de port d'au moins 1 mois.

8. Senofilcon A pour utilisation dans un procédé pour l'atténuation de symptômes de sécheresse oculaire associée aux lentilles de contact selon la revendication 1, ledit patient nécessitant l'atténuation de symptômes d'une évaporation excessive de larmes.

9. Senofilcon A pour utilisation dans un procédé pour l'atténuation de symptômes de sécheresse oculaire associée aux lentilles de contact selon la revendication 1, ledit patient nécessitant l'atténuation de symptômes d'une déficience en larmes aqueuses non-Sjogren.

10. Polymère lubrifiant pour utilisation dans un procédé pour l'atténuation de symptômes de sécheresse oculaire associée aux lentilles de contact comprenant la mise en contact de la surface de l'oeil d'un patient nécessitant l'atténuation de symptômes pour la sécheresse oculaire associée aux lentilles de contact avec une lentille de contact comprenant un module inférieur à 827 kPa (120 psi) et au moins 5 % d'au moins un polymère lubrifiant, ledit polymère lubrifiant comprenant des monomères qui sont solubles lorsque 10 % en poids du monomère sont mélangés dans de l'eau à température ambiante.

11. Polymère lubrifiant pour utilisation dans un procédé pour l'atténuation de symptômes de sécheresse oculaire associée aux lentilles de contact selon la revendication 10, ladite lentille de contact comprenant au moins un composant contenant une silicone.

12. Polymère lubrifiant pour utilisation dans un procédé pour l'atténuation de symptômes de sécheresse oculaire associée aux lentilles de contact selon la revendication 10, ledit polymère lubrifiant ayant un poids moléculaire moyen en poids d'au moins 50 000 daltons.

13. Polymère lubrifiant pour utilisation dans un procédé pour l'atténuation de symptômes de sécheresse oculaire associée aux lentilles de contact selon la revendication 10, ledit polymère lubrifiant ayant un poids moléculaire moyen en poids d'au moins 100 000 daltons.

14. Polymère lubrifiant pour utilisation dans un procédé pour l'atténuation de symptômes de sécheresse oculaire associée aux lentilles de contact selon la revendication 10, ledit polymère lubrifiant comprenant une teneur en eau d'au moins environ 70 % lorsqu'il est polymérisé et réticulé à une quantité mineure.

15. Polymère lubrifiant pour utilisation dans un procédé pour l'atténuation de symptômes de sécheresse oculaire associée aux lentilles de contact selon la revendication 10, ledit polymère lubrifiant étant choisi dans le groupe constitué de polymères et copolymères hydrophiles de poids moléculaire élevé de polyamides, polylactones, polyimides, polylactames et polyamides fonctionnalisés, polylactones, polyimides et polylactames.

16. Polymère lubrifiant pour utilisation dans un procédé pour l'atténuation de symptômes de sécheresse oculaire associée aux lentilles de contact selon la revendication 10, ledit polymère lubrifiant étant choisi dans le groupe constitué de poly-N-vinylpyrrolidone, poly(N-vinyl-N-méthylacétamide), poly-N-vinyl-2-pipéridone, poly-N-vinyl-2-caprolactame, poly-N-vinyl-3-méthyl-2-caprolactame, poly-N-vinyl-3-méthyl-2-pipéridone, poly-N-vinyl-4-méthyl-2-pipéridone, poly-N-vinyl-4-méthyl-2-caprolactame, poly-N-vinyl-3-éthyl-2-pyrrolidone, et poly-N-vinyl-4,5-diméthyl-2-pyrrolidone, polyvinylimidazole, poly-N-N-diméthylacrylamide, poly(alcool vinylique), poly(oxyde d'éthylène), poly-2-éthyloxazoline, polysaccharides d'héparine, polysaccharides, des mélanges et copolymères (comprenant des copolymères séquencés ou statistiques, ramifiés, multichaîne, ramifiés en peigne ou en forme d'étoile) de ceux-ci.

17. Polymère lubrifiant pour utilisation dans un procédé pour l'atténuation de symptômes de sécheresse oculaire associée aux lentilles de contact selon la revendication 10, ledit polymère lubrifiant comprenant des motifs de répétition dérivés de N-vinylpyrrolidone, N-vinyl-N-méthylacétamide et des mélanges de ceux-ci.

18. Utilisation de Senofilcon A pour la fabrication d'une lentille de contact pour l'atténuation de symptômes de sécheresse oculaire associée aux lentilles de contact par mise en contact de la surface de l'oeil d'un patient nécessitant l'atténuation de symptômes pour la sécheresse oculaire associée aux lentilles de contact avec ladite lentille de contact.

19. Utilisation de la revendication 18 dans laquelle lesdits symptômes comprennent sécheresse, brûlure, piqûre et gêne.

20. Utilisation de la revendication 18 dans laquelle ladite étape de mise en contact comprend un temps de port d'au moins 8 heures.

21. Utilisation de la revendication 18 dans laquelle ladite étape de mise en contact comprend un temps de port d'au moins 12 heures.

22. Utilisation de la revendication 18 dans laquelle ladite étape de mise en contact comprend un temps de port d'au moins 24 heures.

23. Utilisation de la revendication 18 dans laquelle ladite étape de mise en contact comprend un temps de port d'au moins 1 semaine.

24. Utilisation de la revendication 18 dans laquelle ladite étape de mise en contact comprend un temps de port d'au moins 1 mois.

25. Utilisation de la revendication 18 dans laquelle ledit patient nécessite l'atténuation de symptômes d'évaporation excessive des larmes.

26. Utilisation de la revendication 18 dans laquelle ledit patient nécessite l'atténuation de symptômes d'une déficience en larmes aqueuses non-Sjogren.

27. Utilisation d'un polymère lubrifiant pour la fabrication d'une lentille de contact comprenant un module inférieur à 827 kPa (120 psi) et au moins 5 % d'au moins un polymère lubrifiant pour l'atténuation de symptômes de sécheresse oculaire associée aux lentilles de contact par mise en contact de la surface de l'oeil d'un patient nécessitant l'atténuation de symptômes pour la sécheresse oculaire associée aux lentilles de contact avec ladite lentille de contact, ledit polymère lubrifiant comprenant des monomères qui sont solubles lorsque 10 % en poids du monomère sont mélangés dans de l'eau à température ambiante.

28. Utilisation de la revendication 27 dans laquelle ladite lentille de contact comprend au moins un composant contenant une silicone.

29. Utilisation de la revendication 27 dans laquelle ledit polymère lubrifiant a un poids moléculaire moyen en poids d'au moins 50 000 daltons.

30. Utilisation de la revendication 27 dans laquelle ledit polymère lubrifiant a un poids moléculaire moyen en poids d'au moins 100 000 daltons.

31. Utilisation de la revendication 27 dans laquelle ledit polymère lubrifiant comprend une teneur en eau d'au moins 70 % lorsqu'il est polymérisé et réticulé à une quantité mineure.

32. Utilisation de la revendication 27 dans laquelle ledit polymère lubrifiant est choisi dans le groupe constitué de polymères et copolymères hydrophiles de poids moléculaire élevé de polyamides, polylactones, polyimides, polylactames et polyamides fonctionnalisés, polylactones, polyimides et polylactames.

33. Utilisation de la revendication 27 dans laquelle ledit polymère lubrifiant est choisi dans le groupe constitué de poly-N-vinylpyrrolidone, poly(N-vinyl-N-méthylacétamide), poly-N-vinyl-2-pipéridone, poly-N-vinyl-2-caprolactame, poly-N-vinyl-3-méthyl-2-caprolactame, poly-N-vinyl-3-méthyl-2-pipéridone, poly-N-vinyl-4-méthyl-2-pipéridone, poly-N-vinyl-4-méthyl-2-caprolactame, poly-N-vinyl-3-éthyl-2-pyrrolidone, et poly-N-vinyl-4,5-diméthyl-2-pyrrolidone, polyvinylimidazole, poly-N-N-diméthylacrylamide, poly(alcool vinylique), poly(oxyde d'éthylène), poly-2-éthyloxazoline, polysaccharides d'héparine, polysaccharides, des mélanges et copolymères (comprenant des copolymères séquencés ou statistiques, ramifiés, multichaîne, ramifiés en peigne ou en forme d'étoile) de ceux-ci.

34. Utilisation de la revendication 27 dans laquelle ledit polymère lubrifiant comprend des motifs de répétition dérivés de N-vinylpyrrolidone, N-vinyl-N-méthylacétamide et des mélanges de ceux-ci.
